Europäisches Patentamt

European Patent Office

Office européen des brevets

(11) Publication number : **0 564 307 A1**

# (12) EUROPEAN PATENT APPLICATION

(21) Application number : **93400109.0**

(51) Int. Cl.⁵ : **A61L 15/16**

(22) Date of filing : **18.01.93**

(30) Priority : **03.04.92 IL 101492**

(43) Date of publication of application :
**06.10.93 Bulletin 93/40**

(84) Designated Contracting States :
**AT BE CH DE DK ES FR GB GR IE IT LI LU NL PT SE**

(71) Applicant : **Levi, Shalom**
**24/4 Hakotel Hamaaravi Street**
**Beer Sheba 84280 (IL)**

(71) Applicant : **Daren, Stephen**
**10 Hapartizanim Street**
**Nes Ziona (IL)**

(72) Inventor : **Levi, Shalom**
**24/4 Hakotel Hamaaravi Street**
**Beer Sheba 84280 (IL)**
Inventor : **Daren, Stephen**
**10 Hapartizanim Street**
**Nes Ziona (IL)**

(74) Representative : **Phélip, Bruno**
**c/o Cabinet Harlé & Phélip 21, rue de La Rochefoucauld**
**F-75009 Paris (FR)**

(54) **Antiskin rash preparation.**

(57)    Skin irritations due to contact with an excreted body fluid such as urine are avoided by buffering such fluid to a pH within the range of from 4.5 to 6.0 and notably 5.0 to 5.5. Suitable buffering substances or compositions are used for this purpose to which optionally an enzyme inhibitor or suppressor may be added.

There are disclosed diapers, sanitary napkins, pads as well as dry compositions and creams, bearing buffering compositions suitable for this purpose.

Fig.1

EP 0 564 307 A1

## FIELD OF THE INVENTION

This invention relates to a method and means for preventing skin irritation due to the adverse conditions which prevail when body fluids are kept in contact with the skin over extended periods of time. Such conditions commonly occur during use, both with infants and adults of sanitary napkins, pads and the like. Due to the combined effects of moisture, pH, fecal enzymes (especially urease) and bacteria (especially *s. aureus and candida albicans*), nappy rash, dermatitis or vaginitis may be caused.

The invention also relates to improved absorbent matrices such as diapers with which the above afflictions are significantly avoided.

## BACKGROUND OF THE INVENTION

With known disposable diapers a large part of let urine is absorbed by super-absorbent polymers. However, the problem of the combined effects of increased pH and enzymatic activities in diapers and sanitary napkins have only partly been resolved [see, e.g. Davis et al., Pediatric Dermatology, Vol. 6, No. 2, 102-108 (1989)].

It is well known that the optimal pH range for most enzymes and bacteria found in the body is pH 6.0 to 9.0. For urease the maximum activity in aqueous media occurs at pH 6.5 [Biochemistry: A.L. Lehninger, 2nd ed., Worth Publishers, Inc., N.Y., p. 51]. On the other hand, the normal pH for skin health is within the range of from about 4.5 to about 5.5. Above pH 6.0, the skin is attacked, mainly by enzymes and bacteria, and where the pH rises above 7 - also by free ammonia.

Urine contains only a few hundred ppm of ammonia existing predominantly as non-toxic ammonium ions due to the pH of the urine which is usually below pH 7.0 (95% ranges 5.1 to 6.8). However, when the pH of the urine rises above pH 7.0, ammonium ions of the urine are converted into free ammonia, which is toxic and an irritant to the skin. Urine contains relatively large quantities of urea (about 20 g/l). Human feces bacteria as well as bacteria present on the skin and in excreted body fluids such as sweat, menstrual blood and smegma contain i.a. the enzyme urease which decomposes urea and thereby liberates ammonia.

This phenomenon occurs particularly vehemently in cases in which skin portions of the body are diapered or padded. Under such conditions there occurs intimate contact between urine and feces whereupon urea decomposes to ammonia and the pH of the urine starts to rise. As the pH rises toward pH 7.0 and above, the activities of fecal proteases and lipases and bacteria, which are pH sensitive, increase rapidly. The bacteria excrete more urease and the damaging cycle continues [Griffith, D.P. et al., The J. of Urology, Vol. 119, 9-15 (1978)]. These enzymatic and bacterial activities have been identified as the main cause of diaper dermatitis.

A similar problem arises with sanitary napkins upon contact between urine and menstrual blood.

## THE PRIOR ART

In order to break the damaging cycle at its onset, it is desirable to suppress the initial activity of the urease and bacteria, and thereby prevent the formation of ammonia and the consequent rise of the pH. The core of disposable diapers contains as a rule an absorbing gel which retains the urine and thereby achieves some control of urine pH [Campbell, R.L. et al., J. Amer. Acad. Dermatol., Vol. 17, No. 6, 978-997 (1987)], and also partial separation of the urine from the feces. However, the sodium salt of polyacrylic acid, from which most of the gellants are prepared has a pH of approximately 6.5, which is still very conducive to enzymatic and bacterial activities in general and urease activity in particular. Furthermore, since the gel is insoluble, its buffering control is restricted to that part of the urine which diffuses into the gel and is retained therein, while any non-absorbed urine is not affected. Accordingly, statistical clinical evaluation of the performance of these so-called super-absorbent diapers showed only small, albeit significant, decreases in the incidence of diaper rash [Campbell, R.L. et al., J. Am. Acad. Dermatol., 17, 6 (1987), p. 984; Davis, J.A. et al., Pediat. Dermatol., Vol 6, 2, 102-108 (1989)].

Deactivation of urease by synthetic compounds has been proposed by several workers. U.S. 4,517,007 discloses the use of phosphoramides for the inhibition of urease in urea organophosphate-based fertilizers. The use of these substances in diapers is also suggested but no information regarding their performance or toxicity is given.

Coppi et al. [Arzneim-Forsch. (Drug. Res.) 20, Nr. 3 (1970), 384-386] demonstrated in hyperammonamic patients the *in vivo* deactivation of urease by acetohydroxamic acid and the *in vitro* deactivation of urease by ascorbic acid. Other urease inhibitors include heavy metals such as zinc, iron, molybdenum and nickel. For example, U.S. 4,556,560 discloses the slow release of water-soluble zinc salts from an absorbent material.

Ascorbic acid is well known for its beneficial effects as a skin-nourishing agent, and often appears in cosmetic formulations. Both ascorbic acid and phosphoric acid have been used in conjunction with iron II compounds as deodorants in diapers.

JP 58/104276 and JP 57/11997 describe the use of hydroxamic acid and its salts in conjunction with cationic surfactants in disposable diapers, and claims to prevent skin rashes caused by ammonia.

U.S. 3,567,820 teaches incorporating organic cation exchange resins in an ointment for application to skin afflicted with ammonia dermatitis, or diaper rash. The purpose of such a resin is to remove ammonium ions present in urine or ammonia produced *in situ* by bacteriological or enzymatic activity.

WO 81/01643 describes the use of natural or synthetic zeolites as ion exchange resins, to remove ammonia from urine. This reference suggests the addition of a buffer in the pH range, "preferably", "approximately" or "near" 7. Such buffers could include disodium hydrogen phosphate and boric acid buffers in the pH range 6 to 8. However, as will be detailed hereinafter, this is just the pH range to be avoided.

EP 202,126 and EP 202,127 teach the inclusion of low molecular weight organic and inorganic acids in localized regions of the diaper with the purpose of maintaining the skin pH within the range of from about 4.0 to about 5.5, and for this the pH of the urine must be reduced to even lower values. Operating according to EP 202,127 has thus the considerable disadvantage of causing the skin to be in contact with acidic urine at pH 3.0 to 4.0. Since the normal skin pH is within the narrow range of from about 4.5 to about 5.5, these lower pHs dramatically alter the physiological conditions of the skin and may lead to skin irritation.

EP 311,344 teaches to incorporate non-volatile antimicrobial agents in diapers. Such an incorporation may actually be deleterious to the skin, when large populations of microorganisms are present. Many antimicrobial agents act on bacteria by breaking down the cell walls, thereby releasing the content of the cells, including urease. Thus, urease-containing bacteria, which are present in feces and on the skin, will actually increase the concentration of urease in urine, when such antimicrobial agents are included.

## SUMMARY OF THE INVENTION

It has been found, in accordance with the invention, that it is possible to prevent, or at least substantially to inhibit, the development of unhealthy skin conditions resulting from contact with excreted body fluids, by inhibiting the activity of enzymes present in such fluids, e.g. urease in case of urine. It has further been found that a simple, effective and efficient way to obtain this result is to control the pH of the body fluid.

The present invention is based, i.a., on the surprising observation that, as distinct from aqueous solutions in which the onset of significant activity of the enzyme urease is known to be within the pH range of from about 4.0 to about 5.0, in urine the onset of significant enzymatic activity is at a higher pH within the range of 5.5 to 6.0, while at the pH range of 4.5 to 5.5 the activity is significantly lower. It is a well known fact that the pH of healthy skin is within the range of 4.5 to 5.5. Due to the above surprising observation made in accordance with the present invention, the control of harmful enzymes occurs at a pH range which essentially coincides with that of healthy skin.

By one aspect the invention provides a method of preventing the development of skin rash and other unhealthy skin conditions resulting from contact of the skin with at least one body fluid, comprising maintaining the pH of said at least one body fluid in contact with the skin within the range of from about 4.5 to about 6.0 by adding thereto a non-toxic, skin-tolerable body fluid-soluble buffer.

If desired a urease inhibitor or suppressor may be used in conjunction with said buffer.

By another aspect, the invention provides a formulation for preventing the development of skin rash and other unhealthy skin conditions resulting from contact of the skin with at least one body fluid, comprising a non-toxic, skin-tolerable, body fluid-soluble binary or single substance buffer capable of maintaining the pH of a body fluid within the range of from about 4.5 to about 6.0, together with a suitable non-toxic, skin-tolerable inert carrier.

If desired, the said formulation may also contain a urease inhibitor or suppressor.

In accordance with the invention the above formulation may be in form of a cream comprising said non-toxic, skin-tolerable, body fluid-soluble buffer together with a cream base. If desired, said cream also includes at least one urease inhibitor or suppressor.

By another embodiment the invention provides a solid or liquid soap comprising said non-toxic, skin-tolerable, body fluid-soluble buffer, if desired together with at least one urease inhibitor or suppressor.

By still a further aspect, the invention provides an absorbent matrix bearing a formulation of the kind specified, which absorbent matrix may, for example, be a diaper, a pad or a napkin.

One of the important applications of this invention is the prevention of skin irritation and rashes due to urine, in particular in diapers. In regard to this application the invention affords the great advantage that reduction in skin irritation due to urease activity and to liberated ammonia, is achieved without any risk of skin damage due to low skin pH values below the natural range of from about 4.5 to about 5.5. It is to be noted in this connection that in accordance with the invention this is achieved by adjusting only the pH of the body fluid which comes into contact with the skin, e.g. urine, while the pH of the skin itself remains in the homeostasis range. In this respect, the invention differs significantly from some prior art which seeks to control chemical and biological processes on the skin by reducing the skin pH to below 4.5. It should further be noted that by effectively controlling the pH of a body fluid that comes into contact with the skin and adjusting it to a level within the natural range of skin pH, optimal control of skin conditions is obtained while at the same time external alteration of the skin pH is avoided.

In the performance of the invention, typically a buffer is used for maintaining the pH of the body fluid within the range of from about 4.5 to about 6.0. In addition, according to an embodiment of the invention, a pH stabilizer selected from the group of metal carbonates and oxides that are sparingly soluble at a pH above about 5.0 and begin to dissolve at a pH of about 5.0 is added, whereby the stipulated lower limit of the desired pH range is maintained. Thus, should the pH of the urine decrease to 5.0, the said pH stabilizer dissolves into the urine and prevents a drop of the pH below about 5.0.

The preferred pH range for the purposes of the present invention is from about 5.0 to about 5.5.

Illustrative and non-limitative examples of buffers suitable for the purposes of the present invention are potassium dihydrogen phosphate, ascorbic acid/ascorbic acid salt, citric acid/citric acid salt or citric acid/phosphate buffers.

In consequence of the activity of enzymes in an excreted body fluid, e.g. urease in the case of urine, the pH of the fluid tends to rise. Accordingly, in order to ensure that the pH remains below pH 6 over extended periods of time, an enzyme inhibitor, e.g. urease inhibitor or suppressor in case of urine, is used in conjunction with a buffer.

Examples of urease suppressors or inhibitors are substances selected from the group of organic acids, heavy metal salts, heavy metal oxides, aliphatic hydroxamic acids and their salts, specific examples being ascorbic acid, citric acid, zinc acetate, zinc carbonate, zinc oxide, acetohydroxamic acid.

In accordance with one embodiment of the invention, the buffer components and, if desired, the pH stabilizer, and also if desired, the urease suppressor or inhibitor, all in dry form, are embedded in an absorbent matrix such as a diaper, napkin or pad. The active ingredients may conveniently be first mixed with an inert carrier and then dispersed into the absorbent matrix. Illustrative examples of suitable carriers are talc, natural or synthetic zeolites and clay, e.g. attapulgite. The inert carrier is useful for the purpose of enabling uniform distribution of the active components in the matrix.

According to another embodiment of the invention, the buffer components and, if desired, the pH stabilizer and further if desired, the urease suppressor or inhibitor are applied to the skin as a cream.

## BRIEF DESCRIPTION OF THE DRAWINGS

**Fig. 1** shows the pH dependence of urease activity in urine;
**Fig. 2** is a literature reproduction and shows the pH dependence of urease activity in an aqueous urea solution;
**Fig. 3** shows the rate of ammonia production by urease in an aqueous urea solution and the increase in pH of the aqueous urea solution as a function of time; and
**Fig. 4** shows the rate of ammonia production by urease in urine and the increase in pH of the urine as a function of time.

## EXPERIMENTAL PROCEDURES

Urine was collected daily from four adult subjects and was used within sixteen hours of the collection. When not in use, the urine and all other solutions were stored in a refrigerator at 4°C.

By the very nature of being a biological material, the urine mixture differed each day with respect to the pH, ammonia content and self-buffering. Accordingly, each experiment was performed against a blank from the same urine sample.

The presence of urease in feces is not a well defined quantity. Therefore, for convenience, purchased urease (Sigma, M.Wt. = 482,700, 24,000 $\mu$ molar unit per gram) was used. The amount of urease added to the urine (5 units/50 mls. urine) was chosen so that for every urine sample tested in the absence of a buffer or inhibitor, the pH rose to above pH 7 during one hour. This simulates the conditions which are likely to occur in the case of diaper rash.

The incubation experiments were performed in a thermostated bath at 37.0-38.0°C. The pH of the urine was determined using a combined glass electrode (Metrohm), and the total ammonia using an Orion 9512 electrode. This ammonia electrode is sensitive only to free ammonia. Therefore, since below pH 7 only negligible amounts of free ammonia exist, the ammonium ions were converted to free ammonia by adding 0.25 ml of 10M sodium hydroxide to each urine sample. On sampling the urine, it was found necessary to dilute the samples by a factor of five in distilled water in order to enter the linear range of the ammonia electrode.

In the following examples and tables the term "ammonia" refers to the total content of ammonium ions ($NH_4^+$) and ammonia ($NH_3$), it being understood that below pH 7, little or no free ammonia actually exists in the urine.

## Example 1

### Reactivity of Urease in Urine at Various pH Values

Six stoppered Erlenmeyer flasks with Teflon-coated magnetic bar stirrers were each filled with 50 ml urine (ammonia content 340 ppm., pH = 6.1). Using either phosphate or citrate buffers, the pH of the urine in each flask was adjusted to a different value in the pH range 4 to 11. The flasks were placed in a thermostated bath at 38°C for 15 minutes for equilibration, and then 1 ml. of urease solution (containing 5 units) was added to each flask. After 60 minutes, the flasks were withdrawn from the bath and the final pH and ammonia contents were determined. The activity of the urease was calculated by dividing the increase in the ammonia concentration ($\Delta[NH_3+NH_4^+]$ ppm) by the residence time in the bath after the addition of the urease ($\Delta t$).

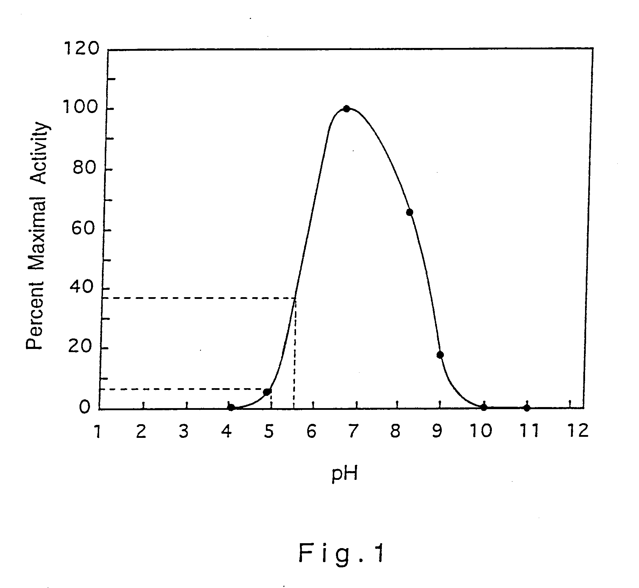

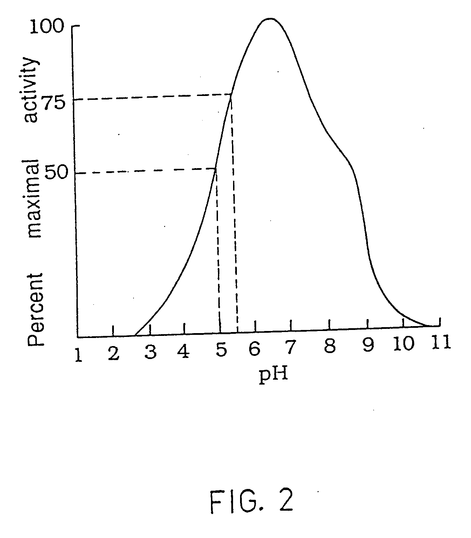

In Fig. 1, $\Delta[NH_3+NH_4^+]/\Delta t$ is expressed as the percent of maximal activity of the urease enzyme in urine and is plotted as a function of the pH for the various samples. As seen, the urease activity increases steeply between pH 5-6.8 while the rate of ammonia formation in the range pH 5-5.5 is only about 5-37% of the peak rate at pH 6.8. In an aqueous urea solution, on the other hand, 50-75% of peak activity is observed at pH 5.0 to 5.5, as shown in Fig. 2 which is reproduced from A.L. Lehninger, "Biochemistry", p 51.

## Example 2

### Reactivity of Urease in Urine and Aqueous Urea

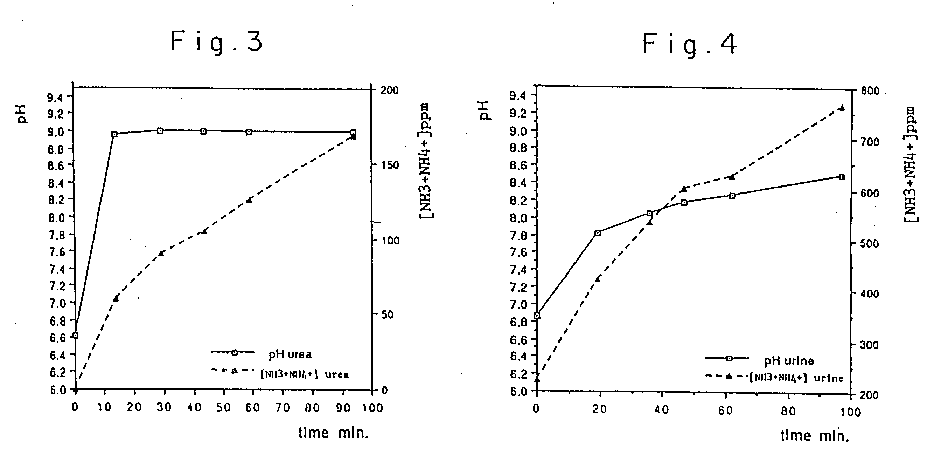

Urease, 5 units (Sigma) were added to 50 mls. urea (British Drug House - BDH 20 g/l) and urine (50 mls.) at 38°C, as in Example 1. Samples were withdrawn periodically and tested for pH and ammonia concentration. The results are presented in Figs. 3 and 4, respectively. In terms of ammonia production, the urease is about three times more reactive in urine than in aqueous urea. On the other hand, the increase in pH of the urine is less than in aqueous urea showing the self-buffering capacity of urine.

The maximum rate of production of ammonia in urine as given by the slope of the graph [$NH_3+NH_4^+$] ppm vs. time, is in the pH range 6.8 to 7.8.

## Example 3

### Lack of Inhibition by Acids Alone

To 50 ml. samples of urine (pH 6.52, [$NH_3+NH_4^+$] 232 ppm) there were added ascorbic acid (46.5 mg), citric acid (20 mg), maleic acid (23 mg) and lactic acid (80% solution) to give a constant pH 5.5. For comparison, urine alone, and urine containing hydrochloric acid, adjusted to pH 5.7, were also tested. Urease (5 units) was added at 38°C, and after three hours each sample was analyzed for pH and total ammonia. The increase in total ammonia was calculated by: $\Delta[NH_3+NH_4^+]$ = [$NH_3+NH_4^+$] after incubation -[$NH_3+NH_4^+$] in urine before incubation. The results are given in Table I below.

**Table I**

| Added to urine | Initial pH | After three hours | | |
|---|---|---|---|---|
| | | pH | $[NH_3+NH_4^+]$ ppm | $\Delta\ [NH_3+NH_4^+]$ |
| --- | 6.52 | 8.62 | 889 | 657 |
| Ascorbic Acid | 5.5 | 8.45 | 998 | 766 |
| Citric Acid | 5.5 | 8.45 | 960 | 728 |
| Maleic Acid | 5.5 | 8.40 | 924 | 692 |
| 80% Lactic Acid | 5.5 | 8.50 | 998 | 766 |
| Hydrochloric Acid | 5.7 | 8.45 | 960 | 722 |

The acids were equal in behavior, and actually had an accelerating effect on the formation of ammonia.

## Example 4

## Combination of Phosphate Buffer With Acids

To 50 ml. samples of urine (pH 6.0, $[NH_3+NH_4^+]$ = 407 ppm) there was added monopotassium phosphate (MKP) (500 mg) as buffer. This reduced the pH of the urine to 5.45. Various acids were added to further reduce the pH to 5.0. The experiment was conducted as in example 2. The results after three hours are given in Table II.

**Table II**

| Added to urine | Initial pH | After three hours | | |
|---|---|---|---|---|
| | | pH | $[NH_3+NH_4^+]$ ppm | $\Delta\ [NH_3+NH_4^+]$ |
| --- | 6.0 | 8.32 | 1298 | 891 |
| MKP (500 mg) | 5.45 | 5.92 | 953 | 546 |
| MKP + Ascorbic Acid (60 mg) | 5.0 | 5.2 | 475 | 68 |
| MKP + Citric Acid (30 mg) | 5.0 | 5.1 | 440 | 33 |
| MKP + Maleic Acid (30 mg) | 5.0 | 5.12 | 475 | 68 |
| MKP + 80% Lactic Acid (0.02 ml) | 5.0 | 5.25 | 514 | 107 |
| MKP + Hydrochloric Acid | 5.0 | 5.12 | 494 | 87 |

These results are attributable to the further reduction of the pH from 5.45 to 5.00 and to the increase of buffering capacity of the urine solution. This explains the difference between Table I and Table II.

## Example 5

## Inhibiting Effect of Acetohydroxamic Acid and a Buffer

To 50 ml. samples of urine (pH 6.14, $[NH_3+NH_4^+]$ = 500 ppm) MKP (500 mg) was added as buffer. To one sample acetohydroxamic acid (Sigma, 30 mg) was added as well. The experiment was performed as in Example 2. After three hours, the results were as follows:

**Table III**

| Added to urine | Initial pH | After three hours | | |
|---|---|---|---|---|
| | | pH | $[NH_3+NH_4^+]$ppm | $\Delta [NH_3+NH_4^+]$ |
| --- | 6.14 | 8.01 | 1456 | 956 |
| MKP | 5.45 | 5.86 | 718 | 218 |
| MKP + acetohydroxamic acid | 5.45 | 5.55 | 500 | 0 |

The combination of buffer and acetohydroxamic acid totally suppressed the ammonia formation. Reported *in vitro* inhibition in aqueous urea by acetohydroxamic acid alone at similar temperatures and concentrations at pH 7.0 is only 79.3% (Coppi and Bonardi, ibid).

## Example 6

### Inhibiting Effect of Zinc Salts

To 50 ml. samples of urine (pH 5.70, $[NH_3+NH_4^+]$ = 356 ppm) there were added MKP, zinc acetate, zinc carbonate and ascorbic acid in the amounts given below. Urease (5 units) was added, as in Example 1. After three hours the results were as follows:

**Table IV**

| Added to urine | Initial pH | After three hours | | |
|---|---|---|---|---|
| | | pH | $[NH_3+NH_4^+]$ ppm | $\Delta [NH_3+NH_4^+]$ |
| --- | 5.70 | 8.32 | 1281 | 925 |
| MKP (500 mg) | 5.22 | 5.92 | 623 | 267 |
| Zinc Acetate (30 mg) | 5.40 | 6.91 | 675 | 319 |
| MKP (500 mg) + Zinc Acetate (30 mg) | 5.08 | 5.40 | 452 | 96 |
| Zinc Carbonate (30 mg) | 5.73 | 8.08 | 1136 | 780 |
| MKP (500 mg) + Zinc Carbonate (30 mg) | 5.30 | 5.88 | 623 | 267 |
| MKP (500 mg) + Zinc Carbonate (30 mg) + ascorbic acid (30 mg) | 5.10 | 5.52 | 490 | 134 |

As seen, zinc acetate is a strong inhibitor alone (65% inhibition), and in combination with the buffer, MKP, 90% inhibition is achieved. Due to its poor solubility, zinc carbonate is much less effective (16% inhibition). The presence of zinc carbonate is, however, valuable in preventing too low pH values (pH 5.0) from being attained. For example, in the presence of zinc carbonate, ascorbic acid (30 mg) only reduced the pH by 0.12 units relative to MKP alone.

## Example 7

### Talc as a Dispersing Agent

To 50 mls. urine (pH 6.47, $[NH_3+NH_4^+]$ = 169 ppm) there were added, as in the previous examples, zinc acetate (30 mg), talc (500 mg) and talc (500 mg) with zinc acetate (30 mg).
After three hours, the results were as follows:

**Table V**

| Added to urine | Initial pH | After three hours | | |
|---|---|---|---|---|
| | | pH | $[NH_3+NH_4^+]$ ppm | $\Delta\,[NH_3+NH_4^+]$ |
| --- | 6.47 | 8.65 | 1092 | 923 |
| Zinc Acetate | 5.80 | 8.45 | 861 | 692 |
| Talc | 6.47 | 8.65 | 861 | 692 |
| Talc + Zinc Acetate | 5.82 | 8.50 | 665 | 496 |

The talc surprisingly has an inhibiting effect on ammonia formation equal to that, in this particular case, of zinc acetate. No synergism, however, exists between the two, and their combined reduction in ammonia is additive.

## Example 8

### Effects of phosphate, ascorbic, citrate and acetate buffers on urease activity in urine

To 50 ml. samples of urine (pH 6.50, $[NH_3+NH_4^+]$ = 323 ppm) 500 mg (3.85 x 10$^{-3}$M) of MKP and equimolar quantities of the organic acids: ascorbic, citric and acetic were added. The pH of the resulting solutions were adjusted to 5.30 by a few drops of concentrated KOH. Urease (5 units) was added at 37.5°C and after three hours of incubation each sample was analyzed for pH and total ammonia. The results are given in Table VI below.

**Table VI**

| Added to urine | Initial pH | After three hours | | |
|---|---|---|---|---|
| | | pH | $[NH_3+NH_4^+]$ ppm | $\Delta\,[NH_3+NH_4^+]$ |
| --- | 6.50 | 8.15 | 1202 | 879 |
| MKP (500 mg) | 5.30 | 5.50 | 500 | 177 |
| Ascorbic acid | 5.30 | 5.40 | 458 | 135 |
| Citric acid | 5.30 | 5.35 | 438 | 115 |
| Acetic acid | 5.30 | 5.25 | 500 | 177 |

## Example 9

### Effects of Zinc Carbonate and Zinc Oxide as pH Stabilizers in Urine

Four stoppered Erlenmeyer flasks with Teflon coated magnetic bar stirrers were each filled with 50 ml urine (pH = 5.7). The pH of the urine in each flask was adjusted to a value of 4.50 by adding concentrated sulfuric acid. To two flasks (Flask No. 1 and Flask No. 2) 73 mg of zinc carbonate. (M.W. = 125) were added and an equimolar amount, 47 mg, of zinc oxide (M.W. = 81) was added to the other two flasks (Flask No. 3 and Flask No. 4). The flasks were placed in a thermostated bath at 38°C and the pH in each flask was measured one hour and three hours after the beginning of the incubation. In Table VII the pH readings of each flask at both times are presented.

## Table VII

| | Initial pH | pH after 1 hour Flask No. 1 | Flask No. 2 | pH after 3 hours Flask No. 3 | Flask No. 4 |
|---|---|---|---|---|---|
| $ZnCO_3$ | 4.50 | 5.10 | 5.09 | 5.12 | 5.11 |
| ZnO | 4.50 | 5.06 | 5.08 | 5.09 | 5.10 |

**Example 10**

**Activity of Various Formulations**

The following formulations were tested as in Example 1.

| No. | Formulation | |
|---|---|---|
| $F_1$: | Urine alone. pH 6.54 $NH_3+NH_4^+ = 160$ ppm. | |
| $F_2$: | MKP = 500 mg | |
| $F_3$: | MKP = 500 mg | Ascorbic Acid = 30 mg |
| | Zinc acetate = 20 mg | Zinc Carbonate = 20 mg |
| | | Talc = 500 mg |
| $F_4$: | MKP = 500 mg | Ascorbic Acid = 30 mg |
| | Talc = 500 mg | Zinc Carbonate = 40 mg |
| $F_5$: | MKP = 700 mg | |

### Table VIII

| | | After three hours | | |
|---|---|---|---|---|
| | Initial pH | pH | $[NH_3+NH_4^+]$ ppm | $\Delta [NH_3+NH_4^+]$ |
| $F_1$ | 6.54 | 8.38 | 2942 | 2782 |
| $F_2$ | 5.74 | 6.27 | 1171 | 1011 |
| $F_3$ | 5.60 | 6.14 | 1077 | 917 |
| $F_4$ | 5.72 | 6.12 | 1171 | 1011 |
| $F_5$ | 5.66 | 5.95 | 950 | 790 |

This urine sample was unusually reactive and generated 3-4 times more ammonia than the previous samples. Despite this fact, the buffer ($F_2$) gave 64% inhibition, as did formulation $F_4$. Formulation $F_3$ gave 67% inhibition. Increased buffer alone ($F_5$) gave 72% inhibition.

### Example 11

### Activity of Various Formulations

The following formulations were tested as in Example 1.

| No. | Formulation | | |
|---|---|---|---|
| $F_6$: | Urine alone. | pH 6.1 | $NH_3+NH_4^+$ = 340 ppm. |
| $F_7$: | MKP = 700 mg | | |
| $F_8$: | MKP = 500 mg | | |
| | Zinc acetate = 20 mg | | Talc = 500 mg |
| | Ascorbic Acid = 30 mg | | Zinc Carbonate = 20 mg |

**Table IX**

| | | After three hours | | |
|---|---|---|---|---|
| | Initial pH | pH | $[NH_3+NH_4^+]$ ppm | $\Delta\,[NH_3+NH_4^+]$ |
| $F_6$ | 6.1 | 8.5 | 1367 | 1027 |
| $F_7$ | 5.3 | 5.67 | 548 | 208 |
| $F_8$ | 5.0 | 5.50 | 527 | 187 |

The increased amount of MKP (700 mg) gave 80% inhibition. The formulation $F_8$ (570 mg. active compounds) gave 82% inhibition.

### Example 12

### Preparation of Diaper

A typical diaper contains the formulation of the invention in powder form, dispersed immediately below the surface material. Typical powder weight is 1-10 g per each diaper, depending on diaper size.

A powder consisting of a mixture of 500 g of acid-washed attapulgite, 500 g of MKP and 20 g of ascorbic acid was delivered through a hopper in 3 g batches. 300 Diapers were prepared and weighed ten at a time, to show an average weight increase of 30 g/10 diapers, as compared with regular diapers.

The amount of powder used will be proportional to the size of the matrix. Typical weights employed in a sanitary pad are in the range 0.1-1.0 g.

### Example 13

### Preparation of Skin Cream

Into 50 ml. water at 50°C, 2 g of a vinyl alcohol-vinylacetate copolymer (20% acetate) were added slowly with stirring. After dissolution of the polymer there were further added 300 mg ascorbic acid, 50 mg zinc acetate and 550 mg monopotassium phosphate. The solution was allowed to cool to room temperature and then aqueous sodium hydroxide (10 M) was slowly added with stirring until a pH 5.0 was obtained.

To 17.5 g of the above solution were added 10 g lanolin, 8.7 g sesame oil and 6.0 g of hot paraffin wax mixed with 2.5 g hot paraffin oil. The whole mixture was thoroughly mixed until a smooth cream was obtained.

### Claims

1. A formulation for preventing the development of skin rash and other unhealthy skin conditions resulting from contact of the skin with at least one body fluid, comprising a non-toxic, skin-tolerable, body fluid-soluble  binary or single substance buffer selected to maintain the pH within the range of about 4.5 to about 6.0 together with a suitable non-toxic skin-tolerable inert carrier.

2. A formulation according to Claim 1, wherein the pH of the buffer is selected to maintain the pH within the range of about 5.0 to about 5.5.

3. A formulation according to Claim 1 or 2, wherein the buffer is selected from the group of potassium dihydrogen phosphate, ascorbic acid and a salt thereof, citric acid and a salt thereof and citric acid/phosphate buffers.

4. A formulation according to any one of Claims 1 to 3, further comprising a urine-soluble urease inhibitor or suppressor.

5. A formulation according to Claim 4, wherein the urease inhibitor or suppressor is selected from the group of organic acids, non-toxic heavy metal salts, non-toxic heavy metal oxides, aliphatic hydroxamic acids and their salts, and carbonate salts.

6. A formulation according to Claim 4, wherein the urease inhibitor or suppressor is selected from the group of ascorbic acid and citric acid.

7. A formulation according to Claim 5, wherein the urease inhibitor or suppressor is selected from the group of zinc acetate, zinc carbonate, zinc oxide, iron (II), carbonate and iron (III) carbonate.

8. A formulation according to Claim 5, wherein the urease inhibitor or suppressor is an acetohydroxamic acid.

9. A formulation according to any one of Claims 1 to 8, further comprising a pH stabilizer selected from the group of metal salts and oxides which is sparingly soluble at a pH above about 5.0 and begins to dissolve at a pH of about 5.0.

10. A formulation according to any one of Claims 1 to 9, wherein the carrier is selected from the group of talc, natural zeolites, synthetic zeolites and clay.

11. A formulation according to Claim 10, wherein the carrier is attapulgite.

12. A formulation according to any one of Claims 1 to 11, being in cream form.

13. A formulation according to any one of Claims 1 to 11, being in form of a liquid or solid soap.

14. An absorbent matrix bearing a formulation according to any one of Claims 1 to 11.

15. An absorbent matrix according to Claim 14, selected from the group of disposable diapers, sanitary napkins and pads.

Fig.1

FIG. 2

Fig.4

Fig.3

EP 0 564 307 A1

14

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 316 518 (VEREINIGTE PAPIERWERKE AG) * page 5, line 19 - line 45 * --- | 1-6 | A61L15/16 |
| X | EP-A-0 121 952 (TEVIC INTERNATIONAL B.V.) * page 2, line 24 - line 38 * * page 3, line 1 - line 6 * * page 5, line 1 - line 15 * --- | 1-6 | |
| X | GB-A-1 357 731 (ETHICHEM LTD) * page 1, line 67 - line 72 * * page 2, line 10 - line 115 * * examples * --- | 1-6,10, 12-15 | |
| Y | EP-A-0 117 613 (THE PROCTER & GAMBLE COMPANY) * claims * --- | 1-15 | |
| Y | US-A-3 920 015 (JOSEPH S.WORTHAM) * column 1, line 56 - line 65 * * column 2, line 43 - line 68; claims * --- | 1-15 | |
| Y | EP-A-0 348 978 (KIMBERLY-CLARK CORP.) * page 5, line 37 - line 45; claims; example 1 * --- | 1-15 | TECHNICAL FIELDS SEARCHED (Int. Cl.5 ) A61L |
| A | WO-A-8 704 069 (JACOB JOSEPH ET AL.) * claims * --- | 1-15 | |
| A | EP-A-0 413 528 (YU, RUEY J. DR.;VAN SCOTT EUGENE J. DR.) * claims * ----- | 1-15 | |

The present search report has been drawn up for all claims

| Place of search | Date of completion of the search | Examiner |
|---|---|---|
| THE HAGUE | 18 JUNE 1993 | M. ESPINOSA |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

&.................................................................
& : member of the same patent family, corresponding
   document

EPO FORM 1503 03.82 (P0401)